# EUROPÄISCHE PATENTSCHRIFT

(11) **EP 3 372 256 B1**
(45) Veröffentlichungstag und Bekanntmachung des Hinweises auf die Patenterteilung: **29.11.2023**
(21) Anmeldenummer: 17159598.6
(22) Anmeldetag: 07.03.2017
(51) Int. Cl.: A61M 1/00

(54) **VERFAHREN ZUR LUMENERKENNUNG**
LUMEN DETECTION METHOD
PROCÉDÉ DE DÉTECTION DE LUMIÈRE

(43) Veröffentlichungstag der Anmeldung: 12.09.2018
(73) Patentinhaber: BSN medical GmbH, 22761 Hamburg (DE)
(72) Erfinder: Schütz, Patrick, 12159 Berlin (DE); Kaufmann, Steffen, 21357 Barum (DE); Steuer, Merlin, 23858 Reinfeld (DE); Panier, Holger, 22697 Tremsbüttel (DE); Willers, Frank, 23554 Lübeck (DE)
(74) Vertreter: Cohausz & Florack

(56) Entgegenhaltungen:
- WO-A1-2013/117318
- WO-A1-2016/018448
- WO-A2-2008/036360
- DE-A1-102010 032 154

## Beschreibung

### Gegenstand der Erfindung

Die vorliegende Erfindung betrifft ein Verfahren zur Kontrolle eines FluidsystemAbschnitts in einem Unterdrucktherapiesystem, das auf der zeitlich aufgelösten Erfassung von induzierten Druckveränderungen in dem Fluidsystem beruht. Die Erfindung betrifft weiterhin eine Vorrichtung und ein Computerprodukt zur Durchführung dieses Verfahrens.

### Hintergrund der Erfindung

Die lokale Unterdrucktherapie ist ein Verfahren zur Behandlung von Wunden, bei dem ein Unterdruck im Wundraum angelegt wird und dadurch Blut und seröse Flüssigkeiten aus dem Wundraum abgeleitet werden.

Die lokale Unterdrucktherapie wird auch als topische negative Druckbehandlung (TNP = topical negative pressure), negative pressure wound therapy (NPWT), Vakuumtherapie oder Vakuum-assistierte Verschlussbehandlung (VAC = vacuum assisted closure) bezeichnet. Hierbei ist anzumerken, dass kein Vakuum im eigentlichen Sinne, sondern nur ein verminderter atmosphärischer Druck angewendet wird.

Die medizinische Anwendung von Unterdruck, z. B. durch Schröpfen ist seit langem bekannt. Erst die technisch ausgereifte Weiterentwicklung dieses einfachen Prinzips mit computergesteuerter Unterdruckerzeugung führte zu einem Paradigmenwechsel in der Behandlung chronischer oder schwer heilender Wunden.

Die lokale Unterdrucktherapie zur Wundheilung besteht aus einem Wundverschluss (Okklusion) in Kombination mit einer Drainage für Wundsekret und Blut, wobei ein Sog durch Unterdruck aufrechterhalten wird.

Mittels einer Pumpe wird ein kontrollierter, örtlich begrenzter Unterdruck in einer Wunde erzeugt und dadurch der Heilungsprozess in chronischen als auch akuten Wunden verbessert und beschleunigt. Dies geschieht durch das Absaugen von Wundsekret und der damit einhergehenden Entfernung von proteolytischen Enzymen, besonders der Matrix-Metalloproteinasen, sowie einer mechanischen Säuberung der Wunde. Weiterhin wird die Ödembildung reduziert und die Durchblutung der Wunde gesteigert. Dies geschieht zum einen durch direkte Einwirkung des Unterdrucks als auch indirekt durch Ableitung der interstitiellen Flüssigkeit. Weiterhin wird durch den Unterdruck die Neoangiogenese, also die Ausbildung neuer Gefäßstrukturen induziert. Weitere Wirkungen sind die Anregung der Bildung von Granulationsgewebe und die Erhöhung der Zellproliferation, die auch zu einer gesteigerten Epithelialisierung führen. Das lokale Vakuum bewirkt zudem, dass sich die Wundränder einander annähern und damit der Wundschluss erleichtert wird. Außerdem führt die Wundversiegelung zu einer Verringerung des Infektionsrisikos. Insgesamt ist somit eine feuchte Wundbehandlung ohne Stau von Wundexsudat und proteolytischen Enzymen gegeben. Der Unterdruck kann kontinuierlich, intermittierend oder konstant sein. Je nach Durchblutungssituation der betroffenen Stelle (z. B. bei vorhandener peripherer arterieller Verschlusskrankheit) kann der Unterdruck in einem Bereich von 25 mmHg bis hin zu ca. 300 mmHg variiert werden. Im Ergebnis führt die lokale Unterdrucktherapie im klinischen Alltag zu einer erhöhten Wundheilungsrate, einer Reduktion der Hospitalisierungs- und Infektionsrate und bei ausgewählten Indikationen auch zu einer Verringerung der Mortalität.

Die lokale Unterdrucktherapie wird insbesondere eingesetzt bei Geschwüren (z. B. Druckgeschwüren, diabetischen, neuropathischen oder venös bedingten Geschwüren), Verbrennungswunden, traumatischen Wunden, komplexen traumatischen Wunden mit exponierten Sehnen oder Knochen, Wunden mit exponierten Prothesen, dehiszente Bauchwunden, sternalen Wundinfektionen, Wunden mit enterokutaner Fistelbildung, therapieresistenten Wunden, infizierten Wunden, postoperativen Wunden, und Hauttransplantaten.

Ein wichtiger Faktor bei der lokalen Unterdrucktherapie ist die kontrollierte Einstellung des Unterdrucks im Wundraum. Der empfohlene Unterdruck liegt je nach Wundfüllmaterial und Indikation in einem Bereich zwischen 50 und 175 mmHg. Ein zu geringer Unterdruck vermag die positiven Effekt auf die Wundheilung nicht oder nur unzureichend auszulösen, wohingegen ein zu starker Unterdruck den Blutfluss im Gewebe inhibiert und als sehr schmerzhaft empfunden wird.

Aus dem Stand der Technik sind zahlreiche Vorrichtungen zur lokalen Unterdrucktherapie bekannt. Eine übliche Vorrichtung zur lokalen Unterdrucktherapie umfasst eine Saugpumpe (im Stand der Technik auch als "Unterdruckquelle" bezeichnet), einen Sensor für die Drucküberwachung, eine Steuerungseinheit zur Kontrolle der Pumpe, Bedienungs- und Anzeigeelemente, einen Flüssigkeitsaufnahmebehälter zur Aufnahme der aus dem Wundraum abgesaugten Flüssigkeiten, Unterdruckverbindungen, mit denen eine Fluidkommunikation zwischen den einzelnen Komponenten hergestellt werden kann, sowie ein Anschlussmittel für zur Wunde führende Saug- oder Messleitungen.

So beschreibt beispielsweise die WO 2010/005709 A1 eine tragbare Wundtherapievorrichtung mit einer Unterdruckquelle und einer zwischen der Unterdruckquelle und der Wundauflage verlaufenden Saugleitung, bei der ein Kanister zur Aufnahme der abgesaugten Flüssigkeit (sog. Drainagebehälter) zwischengeschaltet ist.

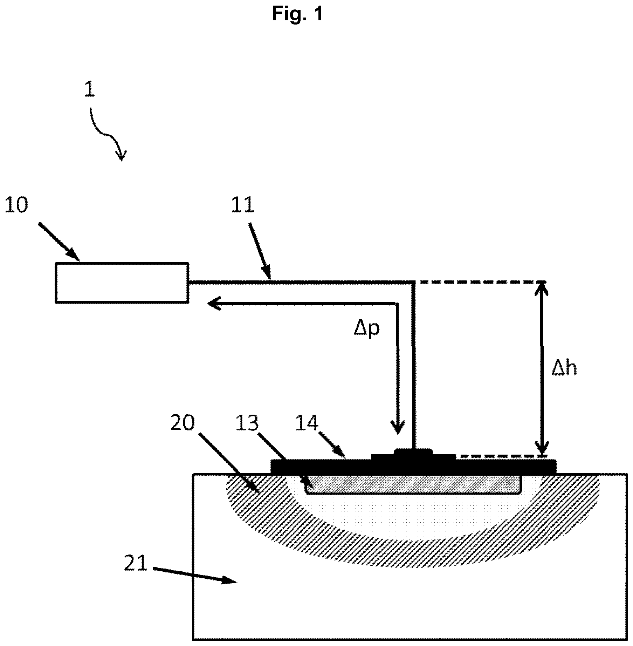

Solche, nur mit einer Saugleitung arbeitende Systeme werden auch als Einlumen-Systeme bezeichnet. Diese Einlumen-Systeme haben den Nachteil, dass der vorrichtungsseitig eingestellte Ziel-Unterdruck nicht dem Unterdruck in der Wunde entspricht, da durch den Höhenunterschied Δh zwischen Unterdruckquelle und der Wunde eine hydrostatischer Druckunterschied Δp entsteht (s. Figur 1). Bei einer exsudatgefüllten Saugleitung und einem Höhenunterschied von 10 cm berechnet sich der Druckunterschied auf ca. 7,4 mmHg. Beachtlicherweise schwankt diese Druckdifferenz zudem während der Therapie durch Eigenbewegungen des Patienten. Angesichts des optimalen Unterdruckbereichs zwischen 50 und 175 mmHg stellen solche große Abweichungen ein erhebliches Problem dar.

Es wurden daher Systeme mit zwei Saugleitungen entwickelt, wobei eine erste Saugleitung zum Absaugen des Exsudats dient (auch "Sauglumen" genannt) und über die zweite, nicht flüssigkeitsgefüllte Saugleitung (sog. "Messlumen") der Unterdruck in der Wunde gemessen wird. Diese Systeme werden auch als Zweilumen-Systeme bezeichnet, wobei durch das zweilumige Schlauchsystem der eingestellte Zieldruck dem Unterdruck in der Wunde entspricht. Durch die bessere Druckregelung und die sofortige Detektion von Blockaden ist hier eine bessere Kontrolle der Therapie möglich.

Diese verbesserte Generation der Unterdrucktherapievorrichtungen ist bereits im Stand der Technik bekannt. So beschreibt die WO 2013/117318 A1 eine Multilumen-Wundtherapievorrichtung mit einem Messlumen 21, einem Instillationslumen 8 und einem Sauglumen 3. Zur breiten Anwendbarkeit können moderne UnterdrucktherapieVorrichtungen sowohl mit Einlumen-Schlauchsystemen als auch mit Zweilumen-Schlauchsystemen verwendet werden. Dies birgt die Gefahr, dass geräteseitig das falsche Schlauchsystem eingestellt wird oder detektiert wird und dementsprechend eine suboptimale oder sogar schädliche Unterdrucktherapie zur Anwendung kommt.

Eine Möglichkeit besteht in der Ausstattung der Schlauchsysteme mit Sensoren, die die Lumen-Information an das Gerät übermitteln. Diese Lösung ist allerdings sehr komplex und angesichts der Schläuche als Einwegartikel auch zu kostenintensiv.

Es besteht daher Bedarf an einem verbesserten Verfahren zum Betreiben einer Unterdruckvorrichtung, die mit einem Ein- und einem Mehrlumenschlauchsystem arbeiten kann.

Aufgabe der Erfindung ist es daher, ein Verfahren zur Kontrolle der Fluidsysteme in Vorrichtungen zur Unterdrucktherapie bereitzustellen, das bezüglich einer der oben genannten Nachteile verbessert ist.

### Zusammenfassung der Erfindung

Diese Aufgabe wird gemäß der Erfindung dadurch gelöst, dass ein Verfahren zur Kontrolle eines Abschnitts in Fluidsystemen eines Unterdrucktherapiesystems zur Erkennung des korrekten Schlauchsystems bereitgestellt wird, wobei dieser Abschnitt von zwei Drucksensoren begrenzt wird, und wobei das Verfahren die folgenden Schritte umfasst:
(a) Aufbauen eines Unterdrucks in dem zu kontrollierenden Abschnitt des Fluidsystems;
(b) Generierung einer Druckveränderung in dem Fluidsystem;
(c) Detektion der Druckveränderung durch die zwei Drucksensoren;
(d) Auswertung der durch die Sensoren erfassten Druckveränderung hinsichtlich ihrer Zeitdifferenz und/oder Größe und/oder ihrer Form;
(e) Steuerung der Vorrichtung zur Unterdrucktherapie in Abhängigkeit von der in Schritt (d) vorgenommenen Auswertung,
wobei
durch die im Verfahrensschritt (c) gemessene Zeitdifferenz oder durch die Druckdifferenz bei einer vorgegebenen Zeitdifferenz zwischen der Messung der beiden Drucksensoren die Länge des Abschnitts im Fluidsystem erfassbar ist, und
durch die im Verfahrensschritt (c) gemessene Zeit- oder Druckdifferenz die Lumenanzahl des Schlauchsystems detektierbar ist, und
wobei
abhängig von der Lumenanzahl die Vorrichtung (1) hinsichtlich folgender Eigenschaft gesteuert wird:
   Erkennung des korrekten Schlauchsystems.

Spezifische Ausgestaltungen der Erfindung sind Gegenstand weiterer abhängiger und unabhängiger Ansprüche.

Das erfindungsgemäße Verfahren vereinigt mehrere entscheidende Vorteile gegenüber den aus dem Stand der Technik bekannten Verfahren.

Durch das erfindungsgemäße Verfahren kann komplett auf eine externe Erkennung der Lumenanzahl verzichtet werden, da die bereits für die Therapie benötigten internen Drucksensoren genutzt werden können.

Da das Verfahren direkt die Länge des Fluidsystems zwischen den Sensoren misst, stellt es eine sichere und fehlerfreie Messung dieser für die Therapie hochrelevanten Komponente dar.

Bei dem erfindungsgemäßen Verfahren handelt es sich um ein einfaches Verfahren, das von der Vorrichtung autark durchgeführt werden kann. Die bevorzugte Durchführung zu Therapiebeginn stellt keine Beeinträchtigung der eigentlichen Therapie dar. Als Vorabtest kann das Verfahren im Rahmen einer Sicherheitsroutine implementiert werden. Hierbei kann geeigneterweise auch das ordnungsgemäße Funktionieren der Drucksensoren und des Belüftungsventils kontrolliert werden (z. B. als Power-On-Self-Test).

Durch die Möglichkeit, weitere Parameter der Druckveränderung auszuwerten, können noch zusätzlich Aussagen über das Fluidsystem getroffen werden:
- So können Engpässe im Fluidsystem detektiert werden. Üblicherweise bestehen die Schläuche eines Unterdrucksystems aus einem weichen, flexiblen Material und sind somit anfällig für Lumenverengungen, bzw. durch Abknicken oder Aufliegen. So werden Engpässe bei gleicher Zeitdifferenz in der Sensordetektion eine andere Drucksignalform an dem hinter der Verengung liegenden Drucksensor ergeben.
- Zudem wäre auch eine komplette Blockade des Fluidsystems einfach detektierbar, insofern die Druckveränderung den zweiten Sensor nicht mehr erreicht.
- Weiterhin können auch im Fluidsystem vorhandene Undichtigkeiten auf diese Weise erkannt werden.

Bei einem zweilumigen System, bei dem die Druckveränderung über die den Wundraum abdichtende Wundauflage weitergegeben wird, können gegebenenfalls auch Aussagen über die Verhältnisse im Wundraum getroffen werden, wie bspw. über das Auftreten und das Ausmaß einer Undichtigkeit oder das Volumen des Wundraums.

Das Verfahren benötigt keine zusätzlichen aufwändigen Gerätekomponenten, sondern kann im Wesentlichen mit den herkömmlichen, entsprechend verknüpften Komponenten durchgeführt werden. Es ist somit mit der bisher verwendeten Gerätetechnologie weitestgehend kompatibel.

Durch einfache Anpassung des Verfahrens bezüglich Drucksensor, Ventil und Steuerungseinheit kann es gezielt an die therapeutischen Erfordernisse angepasst werden.

Aufgrund der vorabgenannten Vorteile eignet sich das Verfahren nicht nur für einen Einsatz in der Unterdruckwundtherapie, sondern es kann bei allen Unterdruck-Fluidsystemen eine Kontrolle von Systemabschnitten erlauben.

### Die Erfindung im Einzelnen

Die Erfindung beinhaltet somit ein mehrstufiges Verfahren, durch das der von zwei Drucksensoren begrenzte Abschnitt kontrolliert wird. Dieser Abschnitt wird primär hinsichtlich seiner Länge kontrolliert. Durch das erfindungsgemäße Verfahren wird somit eine Längenmessung von Fluidsystemen, also bevorzugt von Schlauchleitungen und/oder Rohrleitungen durchgeführt.

In einer bevorzugten Ausführungsform umfasst das Fluidsystem Schlauchleitungen. Diese sind bevorzugt als flüssigkeits- und gasdichte Schläuche ausgebildet. Zweckmäßigerweise ist das Schlauchsystem aus einem Kunststoffmaterial, bzw. einem flexiblen Elastomer- oder Polymermaterial, wie z. B. Silikon gefertigt. Es haben sich dabei besonders flach und biegsam ausgebildete Schläuche bewährt, da diese dem Patienten, wenn er darauf zu liegen kommt, weniger Schmerzen bzw. Druckstellen bereiten.

In einer besonders bevorzugten Ausführungsform umfasst der zu kontrollierende Abschnitt des Fluidsystems zwei unterschiedliche Kompartimente: Das erste Kompartiment beinhaltet die innerhalb der Vorrichtung vorhandenen Abschnitte, die entweder als Schlauchleitung oder Rohrleitung ausgelegt sind. Das zweite Kompartiment ist das außerhalb der Vorrichtung vorgesehene Einlumen, Zweilumen- oder Multilumen-Schlauchsystem, das bevorzugterweise auch den Wundverschluss mit umfasst. Da das geräteseitige erste Kompartiment üblicherweise fest installiert ist, liefert das Verfahren im Wesentlichen eine Aussage über das zweite Kompartiment. Allerdings wird durch das erfindungsgemäße Messverfahren der gesamte Abschnitt des Fluidsystems zwischen den Drucksensoren erfasst und es können somit auch Abweichungen oder Störungen im ersten Kompartiment ermittelt werden.

Voraussetzung für das Messverfahren ist, dass in dem zu kontrollierenden Abschnitt des Fluidsystems ein Unterdruck vorliegt wird. Bei der bevorzugten Kontrolle des externen Schlauchsystems (d. h. des zweiten Kompartiments) bedeutet dies, dass das Fluidsystem in dem zu kontrollierenden Abschnitt geschlossen ist. Dies geschieht zweckmäßigerweise durch Anschluss des Schlauchsystems an der den Wundraum dicht abschließenden Wundauflage (Okklusion). Alternativ können die wundseitigen Enden der Schlauchsysteme verschlossen sein (bei einem Einlumensystem) oder miteinander gekoppelt sein (bei einem Zweilumensystem).

In bevorzugter Weise wird der Unterdruck in Schritt (a) so aufgebaut, dass er als stabiler, d. h. über die Zeit im Wesentlichen unveränderter Unterdruck vorliegt. Damit wird eine störungsfreie Messung erleichtert.

In bevorzugter Weise wird die Unterdruckquelle, die bevorzugt eine Unterdruckpumpe ist, vor Beginn der Messung, also vor der Druckänderung gemäß Verfahrensschritt (b) ausgeschaltet, um einen störungsfreien Ablauf der Messung zu ermöglichen. Die Zeitspanne zwischen dem Abschalten der Pumpe und dem Beginn der Messung, also der beginnenden Detektion des Drucks durch die beiden Sensoren wird als Stabilisierungsphase bezeichnet. Die Stabilisierungsphase sollte einerseits so lange dauern, dass sich ein konstanter Unterdruck einstellt, aber auch zeitlich begrenzt sein, um einem allzu großen Abfall des Unterdrucks entgegenzuwirken. In einer Ausführungsform beträgt die Stabilisierungsphase zwischen 1 und 30 Sekunden, bevorzugt zwischen 2 und 10 Sekunden und besonders bevorzugt 5 Sekunden. Bei besonders kurzen Verbindungen sind auch wesentlich kleinere Zeiten denkbar.

In Schritt (b) wird in dem Fluidsystem eine Druckänderung generiert. In bevorzugter Weise wirkt diese Druckänderung von außerhalb einer der Sensoren auf das Fluidsystem ein.

Gemäß Schritt (c) wird die entsprechende Druckänderung zunächst von diesem nächstgelegenen ersten Sensor erfasst und wandert dann durch das Fluidsystem bis sie nach Durchlauf des zu kontrollierenden Fluidsystemabschnitts von dem hier vorgesehenen zweiten Drucksensor detektiert wird.

Alternativ kann die Druckänderung innerhalb des Fluidsystems an einer bestimmten Stelle (z. B. durch kurzzeitige Ventilöffnung) generiert werden. Die Druckveränderung wandert dann zu den beiden entgegengesetzt gelegenen Sensoren und wird dann entsprechend detektiert.

Die von den beiden Drucksensoren erfassten Signale werden in Schritt (d) ausgewertet, wobei insbesondere die Zeitdifferenz zwischen den Messereignissen von Relevanz ist, da sie ein Maß für die Länge des Fluidsystemabschnitts darstellt. Aber auch weitere Parameter wie die Größe der Druckveränderung oder die jeweilige Form (insbesondere bei pulsförmigen Druckveränderungen) können erfasst und ausgewertet werden.

In Abhängigkeit von der in Schritt (d) vorgenommenen Auswertung wird die Vorrichtung angesteuert, so dass sie bspw. zwischen einem Einlumenbetrieb oder einem Zweilumenbetrieb umschaltet.

In bevorzugter Weise ist der zu kontrollierende Abschnitt des Fluidsystems ein Schlauchsystem. Dieses ist in bevorzugter Weise ein Schlauchsystem, das von dem Gehäuseteil der Vorrichtung zu dem Körper führt. Wie bereits ausgeführt, gibt es hier Einlumen- und Zweilumensysteme und mit dem erfindungsgemäßen Verfahren kann durch die Vorrichtung detektiert werden, welches der beiden Schlauchsysteme hier an die Vorrichtung angeschlossen ist. In besonders bevorzugter Weise sind bei einem Zweilumensystem die Innendurchmesser von Saugschlauch und Messschlauch identisch oder unterscheiden sich nur geringfügig, d. h. weniger als ± 25%, bevorzugt weniger als ± 15% und besonders bevorzugt weniger als ± 7.5% voneinander. Bei einem ähnlichen oder identischen Schlauchinnendurchmesser werden Druckveränderungen unverändert weitergegeben und die Zeitabhängigkeit der Druckveränderung ist direkt proportional zur Länge des Schlauchabschnitts zwischen den Drucksensoren.

Bei einem Fluidsystemabschnitt mit abweichendem Innenquerschnitt im System (also z. B. ein Teilabschnitt mit großem Innendurchmesser und ein Teilabschnitt mit kleinem Innendurchmesser) ist es vorteilhaft, wenn die unterschiedlichen Innenquerschnitte bekannt sind. Dadurch kann die Länge des Fluidsystemabschnitts genauer ermittelt werden.

In einer bevorzugten Ausführungsform handelt es sich bei der Druckveränderung um eine einstufige Druckveränderung. Dies bedeutet, dass ausgehend von dem Basis-Unterdruck, wie er im Schritt (a) des Verfahrens erzeugt wurde, der Unterdruck entweder vergrößert wird, bis er einen zweiten höheren Unterdruckwert erreicht oder umgekehrt der Unterdruck verringert wird, bis ein zweiter geringerer Unterdruck erreicht wird. Im ersten Falle kann dies beispielsweise durch ein Starten der Saugpumpe oder bei laufender Pumpe durch eine (kurzzeitige) Erhöhung der Pumpleistung erreicht werden. Im zweiten bevorzugten Fall kann durch eine Ventilöffnung der Unterdruck schnell in kontrollierter Weise reduziert werden.

Die einstufige Druckveränderung kann hier jede dem Fachmann geläufige Form annehmen. So kann es sich hierbei beispielsweise um eine lineare Druckveränderung oder kurvenförmige Druckveränderung handeln. Die kurvenförmige Druckveränderung kann wiederum sinusförmig oder exponentiell sein.

In einer alternativen Ausführungsform der Erfindung handelt es sich bei der Druckveränderung um eine pulsförmige Druckveränderung. Diese ist bevorzugt ausgewählt ist aus der Gruppe bestehend aus Rechteckpuls, Dreieckpuls, Sägezahnpuls, und sinusförmiger Puls. Besonders bevorzugt ist hierbei eine pulsförmige Druckerniedrigung und insbesondere handelt es sich hierbei um einen Stufenförmigen Druckerniedrigungspuls.

In einer Ausführungsform der Erfindung hat der generierte Druckveränderungspuls eine Pulsdauer von zwischen 5 und 500 ms, bevorzugt von zwischen 10 und 200 ms, besonders bevorzugt von zwischen 15 und 100 ms, und insbesondere bevorzugt von zwischen 20 und 40 ms.

In einer bevorzugten Ausführungsform der Erfindung erfolgt bei dem Verfahren die einstufige oder pulsförmige Druckerniedrigung durch transiente Öffnung eines Ventils.

In besonders bevorzugter Weise ist dieses Ventil distal der Drucksensoren an das Fluidsystem angeschlossen. Hierzu kann beispielsweise ein geräteseitig vorgesehenes Belüftungsventil verwendet werden. Solche Belüftungsventile sind üblicherweise mit dem Messlumen verbunden, um den Unterdruck bei Überschreiten des oberen Druckschwellenwerts abzusenken oder für das zyklische Freispülen der Saugleitung.

In einer alternativen Ausführungsform kann die transiente Druckerniedrigung durch eine lokale Kompression des Fluidsystemabschnitts erfolgen. So kann beispielweise an einer definierten Stelle des Fluidsystemabschnitts der Schlauch soweit komprimiert werden, dass hier eine messbare Druckerniedrigung in dem Fluidsystem induziert wird. Hierzu sind jegliche dem Fachmann bekannten Vorrichtungen zur Schlauchkompression einsetzbar. In einer bevorzugten Ausführungsform handelt es sich hierbei um eine Exzenterscheibe, die durch ihre Drehung eine in Ausmaß und Dauer definierte Kompression des anliegenden Schlauchsystems erlaubt.

In einer weiteren Ausführungsform der Erfindung weist die Druckveränderung, die bevorzugt eine Druckerniedrigung ΔP_{red.} ist, einen Wert zwischen 0,01 mmHg und 300 mmHg auf, bevorzugt einen Wert zwischen 0,1 mmHg und 50 mmHg und besonders bevorzugt einen Wert zwischen 0,4 mmHg und 2 mmHg.

Erfindungsgemäß ist bei dem Verfahren durch die im Verfahrensschritt (c) gemessene Zeitdifferenz die Länge des Abschnitts im Fluidsystem zwischen den Drucksensoren erfassbar.

Alternativ kann eine definierte Zeitspanne zwischen der Messung des ersten und der Messung des zweiten Drucksensors vorgegeben werden und aus den beiden DruckMesswerten, die zu Beginn und zum Ende der definierten Zeitspanne erhoben werden, wird dann eine Druckdifferenz abgeleitet, aus der dann die Länge des Abschnitts im Fluidsystem zwischen den Drucksensoren erfassbar ist.

In einer bevorzugten Ausführungsform wird das externe, d. h. zur Wunde führende Schlauchsystem über einen Adapter mit der Unterdruckvorrichtung verbunden. Bei einem Einlumenschlauchsystem wird durch diesen Adapter dann das Messlumen blindgeflanscht, so dass nur noch das Sauglumen aktiv ist. Bei dieser Blindflanschung wird das Messlumen mit dem Drainagebehälter fluidführend verbunden. Dies kann durch Öffnen eines entsprechend lokalisierten Ventils geschehen. Durch diese Verbindung des Messlumens mit dem Drainagebehälter ist das Messlumen über den Drainagebehälter direkt mit dem Sauglumen verbunden und dieser "Kurzschluss" bewirkt eine geräteinterne Verbindung der beiden Lumen. In bevorzugter Weise ist diese geräteinterne Verbindung so ausgelegt, dass sich die Filter zwischen den Drucksensoren befinden. Im Ergebnis ist damit der Abschnitt zwischen den Drucksensoren bei dem 1-Lumensystem weitaus kürzer als bei dem Zweilumensystem, bei dem auch die externen, d.h. von der Unterdruckvorrichtung zur Wunde führenden Schlauchabschnitte dazugehören.

Erfindungsgemäß dient die im Verfahrensschritt (c) gemessene Zeitdifferenz dazu, die Lumenanzahl des Schlauchsystems zu detektieren.

Im Schritt (e) des Verfahrens, wird die Vorrichtung in Abhängigkeit von der Auswertung in Schritt (d) gesteuert, d. h. in ihrem Verhalten bei der Unterdrucktherapie gerichtet beeinflusst. Dabei wird die Vorrichtung hinsichtlich der Erkennung des korrekten Schlauchsystems gesteuert. Ebenfalls beschrieben aber nicht beansprucht wird die Möglichkeit, dass die Vorrichtung hinsichtlich einer oder mehrerer der folgenden Eigenschaften gesteuert wird:
- Einstellungen für Warnsignale
- (De)aktivierung der Möglichkeit zur Lumenspülung mit Luft
- Darstellung des detektierten Lumensystems in einer Anzeigevorrichtung
- Abschalten der Pumpe
- Sperrung der Unterdruckvorrichtung

Je nach Auswertung kann die Vorrichtung direkt ein Warn- oder Informationssignal in optischer und/oder akustischer Weise ausgeben, um beispielsweise auf eine Blockade oder eine Kontamination im Fluidsystem hinzuweisen. Gleichzeitig kann das auslösende Ereignis in einem zu elektronischen Steuerungseinheit gehörenden elektronischen Speicher festgehalten werden. Optional kann zusätzlich eine Meldung über eine Daten-Fernkommunikation abgesetzt werden, wenn die Vorrichtung über eine entsprechende Kommunikationseinrichtung verfügt. Es wäre dann möglich, das medizinische Pflegepersonal und oder ein Servicezentrum des Geräteherstellers auf einen möglichen Defekt aufmerksam zu machen.

Allgemein ergibt sich bei Vorrichtungen zur Unterdrucktherapie das Problem, dass die abgesaugten Körperflüssigkeiten, welche häufig mit infektiösen Komponenten kontaminiert sind, nicht in die Unterdruckquelle gelangen dürfen. Der Eintritt von Körperflüssigkeiten in die Unterdruckquelle kann die Pumpe oder Pumpenbestandteile beschädigen. Dies kann zu einem sofortigen Ausfall der Pumpe führen, so dass die Therapie unterbrochen und ein Einsatzgerät beschafft werden muss. Der Eintritt geringerer Mengen von Flüssigkeit kann zu einer Verkürzung der Lebensdauer der Pumpe führen. Weiterhin besteht die Gefahr, dass die Flüssigkeit durch die Pumpe hindurchgesaugt und bei einem Ausfall der Pumpe in das Innere des Gerätes ausgeblasen wird. Hierbei können Gerätekomponenten beschädigt und mit infektiösen Flüssigkeiten kontaminiert werden. Im ungünstigsten Falle gelangt infizierte Flüssigkeit in die Umgebung. Eine Kontamination im Inneren des Gerätes oder der Umgebung kann geschehen, ohne dass der Benutzer dies bemerkt. Kontaminationen im Inneren der Therapie-Vorrichtung können beispielsweise den Techniker, das das Gerät wartet, oder auch Patienten, das medizinische Personal oder andere in der Umgebung befindliche Personen gefährden.

Weiterhin können je nach Detektion der Lumenanzahl unterschiedliche Alarminduzierende Parameter eingestellt werden.

Bei Detektion eines Einlumensystems wird geräteseitig die Möglichkeit für eine Lumenspülung mit Luft durch das Belüftungsventil des Messlumens deaktiviert, wobei diese Möglichkeit bei einen Zwei- oder Mehrlumensystem aktiviert werden muss. Bei der Ausführungsform, die mit einem Kurzschluss zwischen dem Sauglumen und dem Messlumen einhergeht, kann das Belüftungsventil dann zum Entlüften des Systems benutzt werden, so zum Beispiel wenn die Therapie beendet wird.

In allgemeiner Weise werden durch die Auswertung und die damit verbundene Erkennung des korrekten Schlauchsystems die dazugehörigen Geräteeinstellungen vorgenommen.

Weiterhin kann das von dem Gerät erkannte Schlauchsystem auch in einer Anzeigevorrichtung, wie beispielsweise einem Display angezeigt werden.

Gemäß einer Ausführungsform führt eine Auswertung, die ein kritischen Gerätestatus detektiert, dann zum sofortigen Abschalten der Pumpe und/oder zur Sperrung der Unterdruckvorrichtung für den Benutzer.

### Vorrichtung

Es ist eine weitere Aufgabe der vorliegenden Erfindung, eine verbesserte Vorrichtung zur Unterdrucktherapie zur Verfügung zu stellen.

In einem weiteren Aspekt stellt die Erfindung eine Vorrichtung zur Unterdrucktherapie (im Rahmen der vorliegenden Erfindung auch als "Unterdrucktherapievorrichtung" oder "Vorrichtung" bezeichnet) zur Durchführung des erfindungsgemäßen Verfahrens bereit, die mindestens Folgendes umfasst:
i. eine Unterdruckpumpe;
ii. optional ein Aufnahmebehälter für Flüssigkeit;
iii. mindestens zwei Drucksensoren ;
iv. Steuerbares Belüftungsventil:
v. Anschlussmittel, die sowohl den Anschluss eines einlumigen als auch eines zwei- oder mehrlumigen Schlauchsystems ermöglicht;
vi. und eine elektronische Steuerungseinheit, die an die Unterdruckpumpe, das Belüftungsventil und an die zwei Drucksensoren anschließbar ist.

In einer Ausführungsform handelt es sich bei der Vorrichtung um eine stationäre Vorrichtung. Bevorzugt kann sie aber auch als tragbare Vorrichtung ausgestaltet sein, da sie hierdurch die Mobilität des Patienten fördern oder erst ermöglichen kann. Tragbar bedeutet, dass die Vorrichtung aufgrund ihrer Ausgestaltung und ihres Gewichts für einen Patienten mitnehmbar ist. Eine solche tragbare Vorrichtung verfügt zweckmäßigerweise über eine autonome Energieversorgung, so beispielsweise über eine Batterie oder einen Akku.

### Unterdruckpumpe

Erfindungsgemäß weist die Pumpe einen Einlass zum Ansaugen von Luft, einen Auslass zum Ausblasen von Luft sowie einen mit Einlass und Auslass kommunizierenden Innenraum auf. Der Innenraum kann bei Betätigung der Pumpe wechselseitig sowohl mit dem Einlass als auch mit dem Auslass kommunizieren. Der Auslass kann in eine Öffnung münden, so dass Fluide unmittelbar in die Atmosphäre entlassen werden. Alternativ können stromabwärts des Auslasses weitere den Fluidstrom leitende Komponenten vorhanden sein. Begrifflich sind von dem Pumpenauslass auch nachgeschaltete Leitungsmittel oder Hohlräume erfasst, deren stromabwärts gelegene Öffnung dann in die Umgebung mündet. Hierbei sind auch nachgeschaltete Geräuschdämpfer und/oder Geruchsabsorber mit umfasst.

Als Pumpe zum Erzeugen des Unterdrucks eigenen sich alle für medizinische Anwendungen üblichen Pumpen. Diese sind zumeist elektrisch angetrieben bzw. elektrisch antreibbar. Dazu gehören Verdrängerpumpen, wie z. B. Membran- und Schlauchpumpen. Vorteil dieser Pumpen ist, dass kein Teil der Pumpe mit dem Fluid in Kontakt kommt, wodurch die Kontamination der Pumpe vermindert, die Reinigung erleichtert und deren Lebensdauer verlängert werden. Denkbar und vorteilhaft wäre auch der Einsatz einer Kolbenpumpe oder Kreispumpe. Zweckmäßig sind auch tragbare und/oder batteriebetriebene Miniatur- oder Mikropumpsysteme. Beispielsweise kann eine ausreichend hohe Pumpleistung bei geringen Ausmaßen, geringem Betriebsgeräusch und niedriger Energieaufnahme durch ein piezoelektrisches Element bewerkstelligt werden.

Erfindungsgemäß wird die Pumpe durch die elektronische Steuerungseinheit angesteuert und kontrolliert.

In bevorzugter Weise ist diese Ansaugpumpe so gestaltet, dass sie im Wundraum unterhalb der Wundauflage einen Unterdruck aufbaut, der besonders bevorzugt zwischen 20 und 250 mm Hg liegt. insbesondere ist die Ansaugpumpe fähig, einen Unterdruck von 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240 oder 250 mm Hg aufzubauen.

### Aufnahmebehälter für Flüssigkeit

In einer bevorzugten Ausführungsform weist die Vorrichtung zur Unterdrucktherapie einen Behälter zur Aufnahme von Flüssigkeiten auf (im Stand der Technik auch als "Drainagebehälter" bezeichnet). Dieser nimmt die aus der Wunde abgesaugten Körperflüssigkeiten oder Instillflüssigkeiten auf. Vorzugsweise ist der Behälter ein flüssigkeits- und gasdichtes Gefäß, welches sich bei den in der Unterdruck- und der Instillationstherapie vorherrschenden Druckverhältnissen nicht wesentlich verformt, bevorzugterweise ein Kunststoffbehälter. Aus hygienischen Gründen ist ein solcher Behälter als Einwegbehälter ausgestaltet, d. h. er wird nach einmaligem Gebrauch entsorgt.

Vorteilhafterweise werden herstellerseitig Aufnahmebehälter mit unterschiedlichen Kapazitäten bereitgestellt. Kleine Behälter, beispielsweise mit einem Fassungsvermögen von 100 mL bis 400 mL sind bevorzugt für den mobilen Betrieb einsetzbar, während größere Behälter mit einem Fassungsvolumen von 400 bis 2000 ml für den stationären Betrieb geeignet sind.

In einer Ausführungsform weist der Aufnahmebehälter in seinem Inneren durch Rippen oder Vorsprünge gebildete Bereiche oder miteinander kommunizierende Kammern auf, so dass die im Inneren des Behälters gesammelten Körperflüssigkeiten bei einer Bewegung des Behälters am unkontrollierten Schwappen gehindert werden.

In einer alternativen Ausführungsform weist die Vorrichtung keinen Aufnahmebehälter auf. Dies ist bei Wunden mit geringem Exsudataufkommen vorteilhaft, bei denen das Exsudat in dem Wundverband selber, beispielsweise durch Flüssigkeits-absorbierende Komponenten, aufgenommen und festgehalten wird.

Vorteilhafterweise ist der Aufnahmebehälter zwischen der Unterdruckpumpe und dem Anschluss für die zur Wunde führenden Saugleitung angeordnet. Der Behälter weist dann mindestens einen Zulauf zum Einspeisen der Körperflüssigkeiten und mindestens einen Ausgang zur Beaufschlagung mit Unterdruck auf.

In bevorzugter Weise umfasst die Vorrichtung ein erstes Gehäuseteil mit einer Unterdruckpumpe und ein zweites Gehäuseteil, das den Behälter zur Flüssigkeitsaufnahme bildet und das an dem ersten Gehäuseteil der Vorrichtung manuell befestigbar und manuell lösbar ist.

### Filter

In einer weiteren Ausführungsform weist die Vorrichtung einen oder mehrere Filter auf, die in dem Fluidsystem enthalten sind. So kann ein Filter in dem zum Sauglumen gehörenden Fluidsystem enthalten sein und dort das Eindringen von Flüssigkeit und Keimen in die Pumpe verhindern. Bei Vorrichtungen mit einem Behälter zur Aufnahme von Flüssigkeiten befindet sich der Filter üblicherweise zwischen dem Behälter und dem Pumpeneinlass. Häufig ist der Filter, bei dem es sich um einen Mikrofilter handelt, mit dem Behälter fest verbunden und wird beim Auswechseln des Behälters mit diesem entsorgt. Bei Vorrichtungen ohne Aufnahmebehälter befindet sich der Filter meist in der Verbandkomponente. Der Filter ist so beschaffen, dass Luft hindurchtreten kann, aber Flüssigkeiten oder Feststoffe und auch Bakterien zurückgehalten werden.

In einer bevorzugten Ausführungsform ist der Filter in dem erfindungsgemäßen Anschlussmittel, das bevorzugt ein Adapter ist, enthalten.

Bei dem Filter kann es sich um einen Membranfilter (beispielsweise aus Zellulose-Lagen) oder einem Filter aus porösem Festkörper (beispielsweise ein Keramikmaterial, ein Kunststoffmaterial, ein Metall oder ein Sinterkörper) handeln.

Der Filter kann zudem eine oder mehrere quellfähige Komponenten aufweisen, wie beispielsweise Carboxymethylcellulose, welche bei Eindringen von Flüssigkeit in den Filter durch Aufsaugen und Festhalten der Flüssigkeit den Durchtritt von Flüssigkeit verhindern.

### Drucksensoren

Als Drucksensor ist jeder Sensor denkbar, der zur Bestimmung des Drucks eines Fluides geeignet ist. Relativdrucksensoren messen den Druck im Bezug zum Luftdruck der Umgebung und kommen beispielsweise als piezoresistive Relativdrucksensoren in der Medizintechnik zum Einsatz. Besonders geeignet sind hierbei miniaturisierte elektronische Drucksensoren, die bei einem geeigneten Druckbereich eine geringe Leistungsaufnahme und eine kompakte Bauform aufweisen.

Erfindungsgemäß steht der Drucksensor in Fluidkommunikation mit dem Fluidsystem und kann Druckmesswerte erzeugen. Der Drucksensor muss so ausgelegt sein, dass eine Erzeugung und Weitergabe von Druckmesswerten über eine Kommunikationsverbindung (insbesondere zur elektronischen Steuerungseinheit) möglich ist.

Zweckmäßigerweise ist ein erster Drucksensor im Messlumen und ein weiterer Drucksensor im Sauglumen vorgesehen.

### Steuerbares Belüftungsventil

Bei dem Ventil handelt es sich um eine regelbares, elektrisch zu betätigendes Ventil, das bevorzugt als magnetisches Ventil ausgestaltet ist. Zweckmäßigerweise dient das Ventil dem Belüften oder Spülen des Wundraums und des Schlauchsystems, wenn das Ventil einen Öffnungsimpuls erhält. Dabei ist beispielsweise Umgebungsluft, Spülflüssigkeit oder ein sonstiges Fluid über das Schlauchsystem in den Wundraum zuführbar. Zweckmäßigerweise erfolgt die Zufuhr über einen Filter.

### Anschlussmittel

Erfindungsgemäß weist die Vorrichtung ein Anschlussmittel auf, das sowohl den Anschluss eines einlumigen als auch eines zweilumigen Schlauchsystems ermöglicht.

In bevorzugter Weise handelt es sich bei dem Anschlussmittel um einen Adapter.

### Elektronische Steuerungseinheit

Erfindungsgemäß ist die elektronische Steuerungseinheit mit den verfahrensrelevanten Komponenten, also den Drucksensoren, der Pumpe und dem Ventil so verknüpft, dass sie diese Komponenten ansteuern kann und/oder (Mess)signale von dem Komponenten empfangen kann. Die Kommunikation erfolgt hierbei bevorzugt durch eine direkte elektrische Verbindung, wie ein Stromkabel, ein USB-Kabel, ein Kabel für serielle Datenübertragung oder ein Kabel für parallele Datenübertragung. Alternativ kann die Kommunikation über eine optische Verbindung, wie beispielsweise eine Glasfaserleitung, realisiert werden oder mittels einer Funkverbindung wie beispielsweise einer WLAN- oder Bluetooth-Verbindung.

Bei der elektronischen Steuerungseinheit handelt es sich bevorzugt um eine programmierbare Steuerungseinheit, welche über eine Schnittstelle, beispielsweise über eine USB-Schnittstelle, mit einem externen elektronischen Gerät kommunizieren kann. Mittels der Kommunikationsverbindung mit dem externen Gerät, beispielsweise mit einem tragbaren Computer kann die Steuerungseinheit programmiert werden und/oder es können im Rahmen einer Wartung auf der Steuerungseinheit gespeicherte Betriebsdaten abgefragt werden. Während der Durchführung der Unterdrucktherapie ist das externe elektronische Gerät normalerweise nicht an die Vorrichtung angeschlossen.

Die Steuerungseinheit kann die Pumpe kontrollieren, d. h. sie kann die Pumpe einschalten, abschalten und die Pumpleistung (z. B. über die Pumpenhubfrequenz) steuern. Die Kontrolle der Steuerungseinheit über die erste Pumpe erfolgt insbesondere mittels Steuerungssignalen. Weiter kann die Steuerungseinheit Druckmesswerte von den Drucksensoren empfangen und auf Basis der Druckmesswerte den Zustand des Fluidsystems ermitteln. Weiterhin kann die Steuerungseinheit während der Therapie die Druckmesswerte des Drucksensors im Messlumen erfassen, was eine permanente Überwachung des Unterdrucks im Wundraum durch die Steuerungseinheit erlaubt. Entsprechend kann der Verlauf der Unterdrucktherapie durch die Steuerungseinheit präzise gesteuert werden. Insbesondere kann die Steuerungseinheit bei Erreichen eines voreingestellten oberen Unterdruck-Schwellenwertes im Wundraum ein Signal zum Abschalten der Pumpe ausgeben, um das Erzeugen eines zu hohen Unterdrucks in der Wunde zu vermeiden. Ein zu hoher Unterdruck kann die Wunde schädigen und die Wundheilung behindern. Vorzugsweise wird der Benutzer der Wundtherapievorrichtung durch geeignete Anzeigemittel über den angelegten Unterdruck und/oder den zeitlichen Verlauf der Unterdrucktherapie informiert werden. Mittels geeigneter Eingabemittel wie beispielsweise Tasten, einer Tastatur, einem Touchscreen oder einem Computerinterface können komplexe Programme zur zeitgesteuerten Unterdrucktherapie eingegeben werden. Besonders vorteilhaft für die Wundheilung ist es, die Unterdrucktherapie intermittierend auszuführen. Bei einer intermittierenden Unterdrucktherapie wird die Höhe des applizierten Unterdrucks in regelmäßigen Abständen variiert. So wird der Unterdruck beispielsweise nach Verstreichen einer vorher festgelegten Zeitspanne abgesenkt. Nach Verstreichen einer vorher festgelegten weiteren Zeitspanne wird der Unterdruck wieder erhöht.

Erfindungsgemäß ist die Steuerungseinheit der Vorrichtung derart ausgebildet, dass sie über die Ansteuerung des Belüftungsventils und Empfangen der Signale der mindestens zwei Drucksensoren die Lumenanzahl eines an dem Anschlussmittel angeschlossenen Schlauchsystems erkennt und dass sie im Erkennungsfall den Betrieb der Vorrichtung an die Lumenanzahl des Schlauchsystems anpasst.

Die elektronische Steuerungseinheit umfasst vorzugsweise Bedienungselemente, beispielsweise Tasten oder eine Tastatur, eine Anzeigevorrichtung und bevorzugt einen Touchscreen, mittels denen ein Benutzer die Therapievorrichtung starten, steuern und abschalten kann. Hierbei ist insbesondere vorgesehen, dass die für das Therapieverfahren ausgewählten Zielparameter wie Höhe, Dauer oder ein komplexeres Therapieschema eingeben werden können.

Eine Vorrichtung zur lokalen Unterdrucktherapie ist beispielsweise unter der Bezeichnung "Curasul" von der Firma BSN medical GmbH (Hamburg, Deutschland) kommerziell erhältlich.

### Weitere Komponenten der Vorrichtung

In einer bevorzugten Ausführungsform ist in der Vorrichtung auch eine Stromversorgung untergebracht. Bei der Stromversorgung, die zum Betrieb von elektrischen oder elektronischen Komponenten (beispielsweise Pumpe, Drucksensor, Steuerungseinheit oder Ventil) erforderlich ist, kann es sich insbesondere um ein Netzteil und/oder einen Akku handeln.

In einer weiteren Ausführungsform kann die Vorrichtung zusätzlich auch einen oder mehrere elektronische Flüssigkeitssensoren enthalten. Diese können dazu dienen, Flüssigkeit im Fluidsystem, dem Aufnahmebehälter und/oder der Pumpe zu detektieren. In bevorzugter Weise handelt es sich bei dem Sensor um einen optoelektronischen Sensor. Alternativ kann ein kapazitativer Sensor eingesetzt werden, der auf der Messung der elektrischen Kapazität beruht oder ein konduktiver Sensor, der die Flüssigkeit aufgrund ihrer Leitfähigkeit detektiert.

In einer Ausführungsform umfasst die Vorrichtung zusätzlich einen Instillbehälter zur Bereitstellung einer Wundspüllösung und eine an die Pumpe angeschlossene Instillleitung, mittels derer die Wundspüllösung aus dem Instillbehälter in den Wundraum zugeführt werden kann. Bei dem Instillbehälter kann es sich beispielsweise um einen Kanister oder um einen flüssigkeitsundurchlässigen Beutel handeln. Hierbei können grundsätzlich Mehrwegbehälter oder Einwegbehälter eingesetzt werden. Vorzugsweise ist der Instillbehälter ein Einwegbehälter, insbesondere ein herstellerseitig mit einer sterilen Instillflüssigkeit gefüllter Einwegbehälter. Für die Instillation wird zweckmäßigerweise noch eine zweite Pumpe eingesetzt, die bevorzugt eine Schlauchpumpe ist. Die Steuerung und Synchronisation dieser Pumpe erfolgt über die Steuerungseinheit.

Gemäß einer weiteren Ausführungsform weist die Vorrichtung eine Belüftungsleitung oder eine Spülleitung auf, wobei die Belüftungsleitung oder die Spülleitung in Fluidkommunikation mit dem Wundraum und mit mindestens einem Drucksensor steht.

Unter einer Belüftungsleitung wird hierbei eine zusätzliche, zum Unterdruckverband und in den Wundraum führende Leitung verstanden, mittels derer zur Druckangleichung oder zur Durchspülung des Fluidsystems Umgebungsluft in den Wundraum eingeführt werden kann. Die zugeführte Umgebungsluft sollte dabei durch einen Bakterienfilter geleitet werden, um eine Kontamination des Wundraums mit Keimen auszuschließen. Der Bakterienfilter kann beispielsweise an dem von der Wunde abgewandten Ende der Belüftungs- oder Spülleitung vorhanden sein. Die Belüftungs- oder Spülleitung steht mit ihrem wundseitigen Ende in Fluidkommunikation mit dem Wundraum. Das von der Wunde abgewandte Ende der Belüftungsleitung oder Spülleitung steht über ein regelbares Belüftungsventil oder Spülventil mit der Umgebung in Fluidkommunikation, so dass eine Belüftung oder Durchspülung des Wundraums und der Drainageleitung ermöglicht wird, wenn das Ventil einen Öffnungsimpuls erhält. Bei dem Belüftungsventil oder Spülventil handelt es sich normalerweise um ein elektrisch zu betätigendes Ventil, vorzugsweise um ein magnetisches Ventil, welches mit der Steuerungseinheit kommuniziert. Während des Betriebs der Pumpe kann das magnetische Spülventil, beispielsweise in vorbestimmten Intervallen, von der Steuerungseinheit kurzzeitig, beispielsweise für 50 bis 100 Millisekunden, geöffnet werden.

### Computerprogrammprodukt

In einem weiteren Aspekt umfasst die Erfindung ein Computerprogrammprodukt, welches direkt in eine Speichereinheit geladen werden kann und Softwareabschnitte umfasst, mit denen das Verfahren gemäß einem der Ansprüche ausgeführt werden kann, wenn das Computerprogrammprodukt auf einer Vorrichtung zur Unterdrucktherapie ausgeführt wird.

### DEFINITIONEN

Im Rahmen der Erfindung stellt ein "Unterdrucktherapiesystem" ein System zur Durchführung einer lokalen Unterdrucktherapie (bevorzugt von Wunden) dar. Als solche umfasst sie nicht nur die eigentlichen Vorrichtung zur Unterdrucktherapie, die Pumpe, Drucksensoren, Belüftungsventil, Aufnahmebehälter und Steuerungseinheit umfasst, sondern auch die an das diese Vorrichtung angeschlossenen Schlauchsysteme und in einer Ausführungsform auch den Unterdruckwundverband.

Gemäß der Erfindung ist unter einem "Fluid" sowohl ein gasförmiges Medium wie Luft oder Wasserdampf als auch ein flüssiges Medium wie Wasser, Körperflüssigkeiten oder Spülflüssigkeiten zu verstehen. Hierbei werden auch viskose Flüssigkeiten mit erfasst. Dies steht im Einklang mit der therapeutischen Anwendung, bei der sowohl Luft aus dem Wundraum abgesaugt wird, um den entsprechenden Unterdruck aufzubauen, als auch Flüssigkeiten aus dem Wundraum abgezogen werden. In den von der Wunde zur Vorrichtung führenden Leitungen (Saugleitung oder Unterdruckleitung) können Flüssigkeiten (insbesondere Körperflüssigkeiten), Gase (insbesondere Luft) oder Mischungen aus Luft und Flüssigkeiten vorliegen. Flüssigkeit kann auch in Form sehr feiner Tröpfchen in der zum Gerät hin gesaugten Luft vorhandenen sein. Des Weiteren kann die Luft als feuchte Luft einen hohen Anteil an Wasserdampf enthalten.

Ein "Fluidsystem" gemäß der vorliegenden Erfindung ist ein länglicher Hohlkörper, dessen Länge (in der Regel wesentlich) größer als sein Durchmesser ist und der für den Transport von Fluiden, also Flüssigkeiten und Gasen geeignet ist. Bevorzugt sind hierunter Rohrleitungen oder Schlauchleitungen zu verstehen. Dazwischengeschaltete Komponenten wie Kanister, Anschlusstücke, Muffen oder Winkel sind ebenso Bestandteil des "Fluidsystems".

Im Rahmen der Erfindung bezeichnet der "Unterdruck" einen im Wundraum vorhandenen Druck, der gegenüber dem Umgebungsluftduck als atmosphärischer Luftdruck erniedrigt ist. Der Unterdruck als verminderter atmosphärischer Druck wird häufig auch als negativer Druck bezeichnet. Die Druckdifferenz zwischen dem Unterdruck und dem Umgebungsluftdruck wird im Einklang mit der fachmännischen Nomenklatur im Rahmen der Erfindung in mmHg angegeben (Millimeter Quecksilbersäule). 1 mmHg entspricht 133,322 Pa (Pa) bzw. einem Torr.

Wie auch in der Fachwelt üblich, wird im Rahmen der Anmeldung der Unterdruck als positiver Zahlenwert in mmHg angegeben.

Gemäß der Erfindung ist eine Druckerhöhung eine Vergrößerung des Unterdrucks, also eine Vergrößerung der Druckdifferenz zwischen dem Umgebungsluftdruck und dem Luftdruck im Wundraum. Folglich stellt eine Druckerniedrigung erfindungsgemäß eine Verringerung des Unterdrucks, also eine Verminderung der Druckdifferenz zwischen dem Umgebungsluftdruck und dem Luftdruck im Wundraum dar.

Erfindungsgemäß ist unter dem Begriff der "Behandlung" jegliche Anwendung der erfindungsgemäßen Vorrichtung am Individuum zu verstehen, die dazu dient, die Erkrankung symptomatisch oder kausal zu lindern oder sogar gänzlich zu unterdrücken oder das Voranschreiten der Erkrankung aufzuhalten, zu verzögern oder hinauszuschieben. Das zu behandelnde Individuum kann hier ein Mensch oder ein Tier sein.

In den Patentansprüchen verwendete Begriffe wie "umfassen", "aufweisen", "beinhalten", "enthalten" und dergleichen schließen weitere Elemente oder Schritte nicht aus. Die Verwendung des unbestimmten Artikels schließt eine Mehrzahl nicht aus. Eine einzelne Einrichtung kann die Funktionen mehrerer in den Patentansprüchen genannten Einheiten bzw. Einrichtungen ausführen. In den Patentansprüchen angegebene Bezugszeichen sind nicht als Beschränkungen der eingesetzten Mittel und Schritte anzusehen.

Im Einklang mit der vorangehenden Beschreibung werden die folgenden Ausführungsformen offenbart, die alleine oder in jeglicher Kombination mit dem vorgenannten Ausführungsformen Teil der Erfindung sind.

### BEISPIELE

### FIGURENLEGENDEN

Die Erfindung wird nachfolgend anhand der Figuren näher erläutert ohne die Erfindung auf dieses zu beschränken. Es zeigen:
- Fig. 1:: eine schematische Darstellung einer Ein-Lumen-Unterdrucktherapievorrichtung mit Darstellung der hydrostatischen Druckdifferenz zwischen Ziel-Unterdruck und wundseitig herrschendem Unterdruck.
- Fig. 2:: eine schematische Darstellung einer Einlumen-Unterdruckvorrichtung zur Durchführung des erfindungsgemäßen Verfahrens.
- Fig. 3:: eine schematische Darstellung einer Zweilumen-Unterdruckvorrichtung zur Durchführung des erfindungsgemäßen Verfahrens.
- Fig. 4:: eine schematische Darstellung zur Anordnung der Einzelkomponenten in der Unterdrucktherapievorrichtung.
- Fig. 5:: ein Flussdiagram zu den Verfahrensschritten des erfindungsgemäßen Verfahrens
- Fig. 6:: eine Darstellung der Druckverläufe der beiden Drucksensoren bei einem einlumigen System (oben) mit entsprechender Auswertung (unten) zur Identifizierung des Einlumensystems.
- Fig. 7:: eine Darstellung der Druckverläufe der beiden Drucksensoren bei einem zweilumigen System (oben) mit entsprechender Auswertung (unten) zur Identifizierung des Zweilumensystems.
- Fig. 8:: eine schematische Darstellung des erfindungsgemäßen Messverfahrens mit den einzelnen Messphasen und der Aktivierung der verfahrensrelevanten Komponenten Pumpe, Ventil und Drucksensormessung.

### Detaillierte Beschreibung der Ausführungsbeispiele

- Figur 1: zeigt eine schematische Darstellung einer Ein-Lumen-Unterdrucktherapievorrichtung 1 mit einer Pumpe 10, die über das Sauglumen 11 im Wesentlichen gasdicht mit der Unterdruckwundauflage bestehend aus Vakuumpad 14 und Schaumauflage 13 verbunden ist. Das Vakuumpad 10 dichtet die Wundhöhle 20 allseitig möglichst gasdicht ab und bildet somit einen evakuierbaren Wundraum. Durch einen pumpenseitig vorhandenen Drucksensor (nicht dargestellt) wird der Unterdruck im Sauglumen 11 ermittelt. Durch den Höhenunterschied Δh zwischen Drucksensor und Vakuumpad 14 ergibt sich ein Druckunterschied Δp. Dieser Druckunterschied berechnet sich bei einem flüssigkeitsgefüllten Sauglumen 11 als hydrostatischer Druck durch die Formel: P = ρ • g • h, mit P als Druck, ρ als Dichte, g als Erdbeschleunigung und h als Höhe der Flüssigkeitssäule. Eine Wassersäule von 10 cm ergibt einen Druckunterschied von 0.0981 bar (entspricht 7,4 mm Hg).

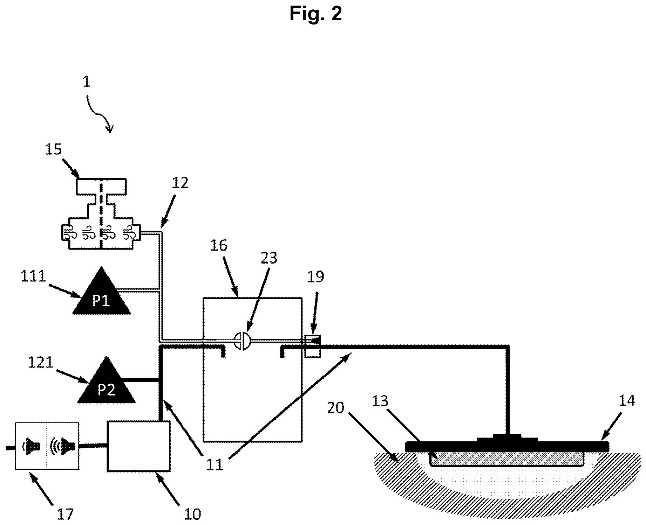

- Fig. 2:: zeigt eine schematische Darstellung einer Einlumen-Unterdruckvorrichtung 1 mit einer Pumpe 10 die über das Sauglumen 11 einen Unterdruck im Wundraum aufbaut und Exsudat in den Drainagebehälter absaugen kann. Das Sauglumen 11 weist einen zwischen Drainagebehälter 16 und Pumpe 10 angebrachten Drucksensor 121 (P2) auf und das Messlumen 12 einen Drucksensor 111 (P1), der zwischen Drainagebehälter 16 und Belüftungsventil 15 angeordnet ist. An der Pumpe 10 ist auslassseitig ein Geräuschreduzierer 17 angeschlossen. Der von dem Drainagebehälter 16 zur Wundauflage führende Schlauch des Sauglumens 11 (rechter Pfeil) ist über einen Adapter 19 mit der Unterdruckvorrichtung verbunden. In diesem Adapter 19 wird das Messlumen 12 blindgeflanscht, so dass durch diesen Adapter ausschließlich ein durchgängiges Sauglumen-Fluidsystem bereitgestellt wird. In dieser Ausführungsform wird das Ventil 23 so eingestellt, das das Messlumen eine Verbindung zum Drainagebehälter aufweist und damit ein Kurzschluss zwischen den gerätseitigen Fluidabschnitten von Sauglumen und Messlumen erfolgt. Bei dem erfindungsgemäßen Messverfahren wird zunächst durch Betätigung der Pumpe 10 ein ausreichender und stabiler Unterdruck im Fluidsystem bestehend aus Sauglumen 11, Drainagebehälter 16 und Wundraum 20 aufgebaut. Nach Abschalten der Pumpe wird dann durch kurzzeitiges Öffnen des Belüftungsventils 15 eine Druckerniedrigung (vulgo eine Verringerung des Unterdrucks) induziert, die als erstes von dem im Messlumen positionierten Drucksensor 111 (P2) detektiert wird. Durch die Blindflanschung des Messlumens 12 im Adapter 19 und gleichzeitige Ventilöffnung 23 kann die Druckveränderung an das Sauglumen und den zweiten Drucksensor 121 (P2) weitergegeben werden. Dieser Drucksensor misst dann nach einer vorgegebenen Zeitspanne einen reduzierten Unterdruck, so dass die Unterdruckvorrichtung nach Auswertung in der Steuerungseinheit (nicht dargestellt) die Anwesenheit eines 1-Lumensystems detektiert.

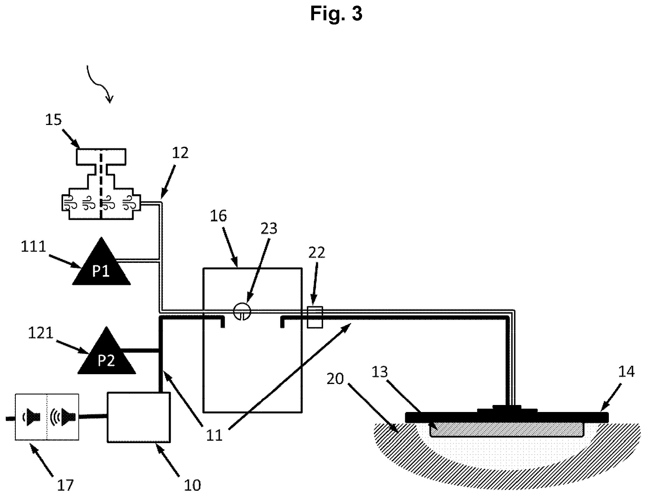

- Fig. 3:: zeigt eine schematische Darstellung einer Zweilumen-Unterdruckvorrichtung 1 mit einer Pumpe 10 die über das Sauglumen 11 einen Unterdruck im Wundraum aufbaut und Exsudat in den Drainagebehälter absaugen kann. Das Sauglumen 11 weist einen zwischen Drainagebehälter 16 und Pumpe 10 angebrachten Drucksensor 121 (P2) auf und das Messlumen 12 einen Drucksensor 111 (P1), der zwischen Drainagebehälter 16 und Belüftungsventil 15 angeordnet ist. An der Pumpe 10 ist auslassseitig ein Geräuschreduzierer 17 angeschlossen. Die von dem Drainagebehälter 16 zur Wundauflage führenden Schläuche des Sauglumens 11 und des Messlumens sind über einen Adapter 22, der bevorzugt das Endstück des externen Doppelschlauchsystems darstellt, mit der Unterdruckvorrichtung verbunden. In dieser Ausführungsform ist das Ventil 23 so eingestellt, dass das Messlumen keine Verbindung zum Drainagebehälter aufweist. Bei dem erfindungsgemäßen Messverfahren wird zunächst durch Betätigung der Pumpe 10 ein ausreichender und stabiler Unterdruck im Fluidsystem bestehend aus Messlumen 12, Sauglumen 11, Drainagebehälter 16 und Wundraum aufgebaut. Nach Abschalten der Pumpe wird dann durch kurzzeitiges Öffnen des Belüftungsventils 15 eine Druckerniedrigung (vulgo eine Verringerung des Unterdrucks) induziert, die als erstes von dem im Messlumen positionierten Drucksensor 111 (P1) detektiert wird. Die Druckveränderung wird dann über den Wundraum 20an das Sauglumen weitergegeben und dann durch den zweiten Drucksensor 121 (P2) detektiert und nach Auswertung in der Steuerungseinheit (nicht dargestellt) als Zweilumensystem detektiert.

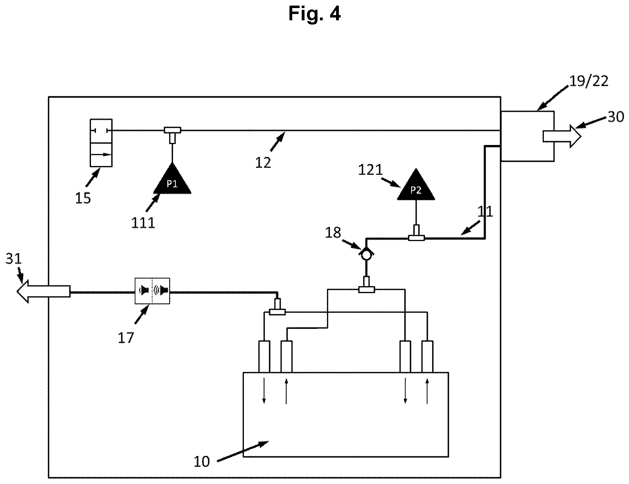

- Fig. 4:: eine schematische Darstellung zur Anordnung der Einzelkomponenten in der Unterdrucktherapievorrichtung, mit der Anschlußmöglichkeit für die Adaptoren 19 bzw. 22. Das Messlumen erstreckt sich zwischen dieser Anschlußmöglichkeit und dem Belüftungsventil 15 und ist mit einem Drucksensor 111 (P1) verbunden. Das Sauglumen erstreckt sich zwischen der Anschlußmöglichkeit und der Saugpumpe 10 mit einem dazwischengeschalteten Drucksensor 121 (2) und einem Kontrollventil 18. Die abgesaugte Luft wird über den Pumpenauslass und einen Geräuschreduzierer durch den Auslass aus der Vorrichtung ausgeleitet.

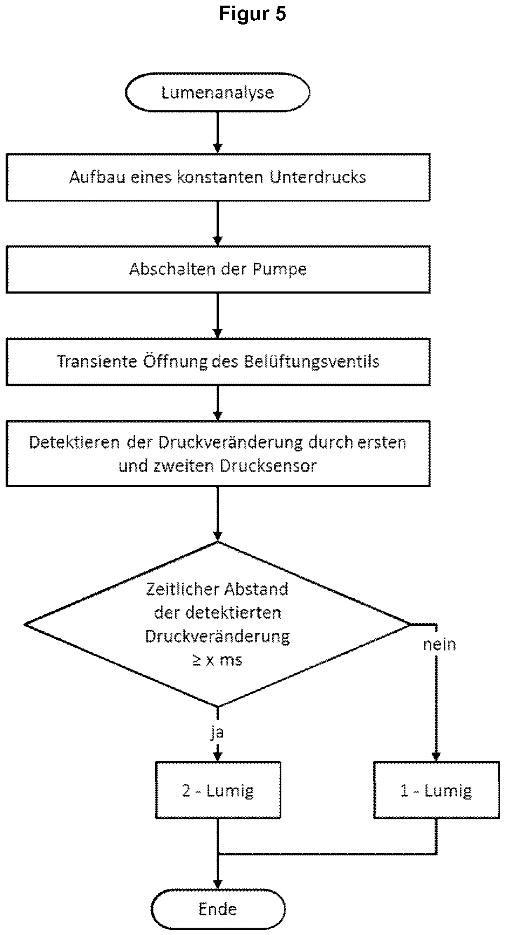

- Fig. 5:: ein Flussdiagram zu den Verfahrensschritten des erfindungsgemäßen Verfahrens zur Lumenanalyse. Nach Aufbau eines konstanten Unterdrucks durch die Pumpe wird diese im zweiten Schritt abgeschaltet. Anschließend wird das Belüftungsventil transient geöffnet und hierdurch ein partieller Abfall des Unterdrucks im Fluidsystem induziert. Dieser Druckabfall wird von dem ersten und dem zweiten Drucksensor detektiert und die der zeitliche Abstand der Druckveränderung am ersten und zweiten Drucksensor ausgewertet. Übersteigt dieser zeitliche Abstand einen vorgegebenen Schwellenwert von x ms, so handelt es sich um ein 2-lumiges System, liegt er darunter, so handelt es sich um ein einlumiges System.

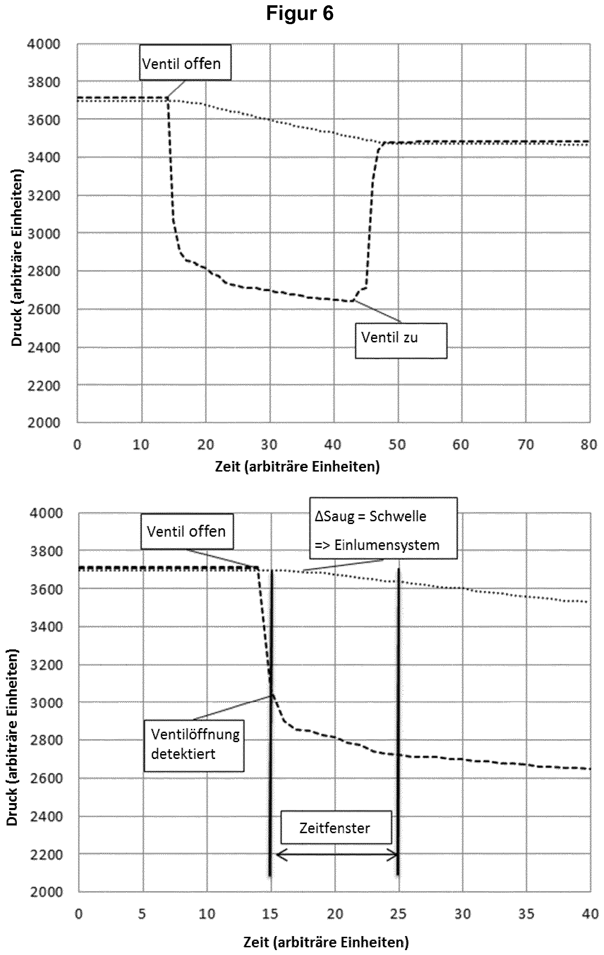

- Fig. 6:: eine Darstellung der Druckverläufe der beiden Drucksensoren über die Zeit bei einem ein-lumigen System (oben) mit entsprechender Auswertung (unten) zur Identifizierung des Einlumensystems. Die am ersten Drucksensor 111 (P1) erhobenen Messdaten sind als gestrichelte Linie dargestellt, die Messdaten des zweiten Drucksensors 121 (P2) hingegen als gepunktete Linie. Bei der vorliegenden Ausführungsform führt die Öffnung des Ventils zu einem starken Abfall des Unterdrucks am Drucksensor 111 (P1), die dann nach Schließen des Ventils durch den intraluminären Druckausgleich wieder ansteigt. Dieser Druckabfall wird an dem Drucksensor 121, der am anderen Ende des Fluidsystemabschnitts angebracht ist, als abgeschwächter linearer Druckabfall detektiert. Bei der Auswertung wird ein Schwellwert für den Druckabfall festgelegt, der dann als Detektion der Ventilöffnung definiert wird und der zu diesem Zeitpunkt am Sensor 121 (P2) gemessene Druck als Initialwert festgehalten. Dieser Initialwert wird mit einem zweiten Wert des Sensors 121 (P2), der nach einem definierten Zeitraum erhoben wird (hier 10 arbiträre Einheiten), verglichen. Entspricht die Differenz ΔSaug einem vorab definierten Schwellenwert, oder ist sie größer als dieser, so handelt es sich um ein Einlumensystem.

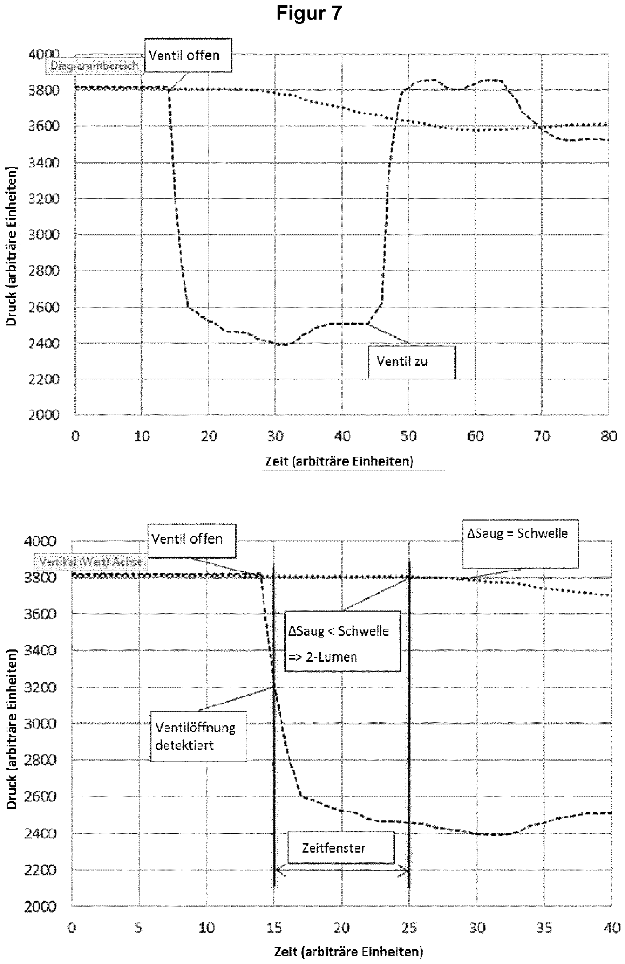

- Fig. 7:: eine Darstellung der Druckverläufe der beiden Drucksensoren bei einem zweilumigen System (oben) mit entsprechender Auswertung (unten) zur Identifizierung des Zweilumensystems. Die am ersten Drucksensor 111 (P1) erhobenen Messdaten sind als gestrichelte Linie dargestellt, die Messdaten des zweiten Drucksensors 121 (P2) hingegen als gepunktete Linie. Die Messung und Auswertung erfolgt wie für Fig. 6 bereits beschrieben. Hier ist zu beachten, dass in der unteren Abbildung nur das Zeitfenster von 0 bis 40 arbiträren Zeiteinheiten dargestellt ist. In dem definierten Zeitfenster von 10 arbiträren Einheiten kommt es aufgrund der längeren Schlauchsystems im zweilumigen System zu keinerlei signifikanten Änderung des Messwerts am Drucksensor 121 (P2). Entsprechend wird dann bei einer Differenz ΔSaug, die kleiner als ein vorab definierter Schwellenwert ist, ein Zweilumen-System erkannt. Beachtlicherweise kann es zudem bei einem Zweilumensystem nach dem Ventilverschluß zudem zu Druckschwankungen am Drucksensor 111 (P1) kommen.

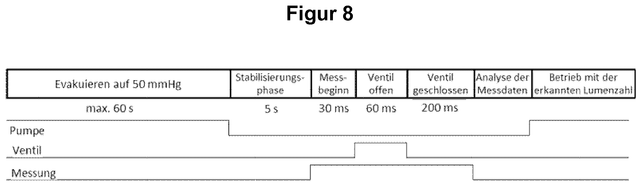

- Fig. 8:: eine schematische Darstellung des erfindungsgemäßen Messverfahrens mit den einzelnen Messphasen. In einem ersten Schritt wird durch Einschalten der Pumpe für einen Zeitraum von max. 60 Sekunden ein Unterdruck von 50 mmHg aufgebaut. Dann wird die Pumpe für die gesamte Zeitdauer des eigentlichen Messverfahrens umfassend Stabilisierungsphase, Messbeginn, Ventilöffnung und Ventilschließung und Analyse der Messdaten ausgestellt, und erst danach wieder aktiviert. Das Messverfahren beginnt mit einer Stabilisierungsphase von 5 sec und dem Beginn der Messung des Unterdrucks durch die beiden Drucksensoren, die von nun an kontinuierlich erfolgt. Nachdem die Drucksensoren für 30 ms Messdaten aufgenommen haben, wird im nächsten Schritt das Belüftungsventil 15 für 30 ms geöffnet. Dadurch strömt Außenluft in das Fluidsystem und der darin vorherrschende Unterdruck wird verringert (als Druckerniedrigung im Sinne der Erfindung bezeichnet). Anschließend wird das Belüftungsventil wieder geschlossen und die Druckveränderung bei geschlossenem Ventil über einen Zeitraum von 300 ms erfasst. Im letzten Schritt erfolgt die Analyse der Messdaten zur Erkennung der Lumenanzahl.

### BEZUGSZEICHEN

- 1: Unterdrucktherapievorrichtung
- 10: Pumpe
- 11: Sauglumen
- 111: Erster Drucksensor
- 12: Messlumen
- 121: Zweiter Drucksensor
- 13: Schaumauflage
- 14: Vakuumpad
- 15: Belüftungsventil
- 16: Drainagebehälter
- 17: Geräuschreduzierer
- 18: Kontrollventil
- 19: Adapter für Anschluß eines einlumigen Schlauchsystems inklusive Filter
- 20: Wundhöhle
- 21: Gewebe
- 22: Adapter für Anschluß eines zweilumigen Schlauchsystems inklusive Filter
- 23: Ventil zum Kurzschließen von Sauglumen und Messlumen
- 30: Verbindung zum Drainagebehälter
- 31: Auslass nach Außen

## Patentansprüche

1. Verfahren zur Kontrolle eines Abschnitts in Fluidsystemen eines Unterdrucktherapiesystems zur Erkennung des korrekten Schlauchsystems, wobei dieser Abschnitt von zwei Drucksensoren (111, 121) begrenzt wird, umfassend die folgenden Schritte:
(a) Aufbauen eines Unterdrucks in dem zu kontrollierenden Abschnitt des Fluidsystems;
(b) Generierung einer Druckveränderung in dem Fluidsystem;
(c) Detektion der Druckveränderung durch die zwei Drucksensoren (111, 121);
(d) Auswertung der durch die Sensoren (111, 121) erfassten Druckveränderung hinsichtlich ihrer Zeitdifferenz und/oder ihrer Größe und/oder ihrer Form;
(e) Steuerung einer Vorrichtung zur Unterdrucktherapie (1) in Abhängigkeit von der in Schritt (d) vorgenommenen Auswertung,
wobei
durch die im Verfahrensschritt (c) gemessene Zeitdifferenz oder durch die Druckdifferenz bei einer vorgegebenen Zeitdifferenz zwischen der Messung der beiden Drucksensoren die Länge des Abschnitts im Fluidsystem erfassbar ist, und
durch die im Verfahrensschritt (c) gemessene Zeit- oder Druckdifferenz die Lumenanzahl des Schlauchsystems detektierbar ist, und
wobei
abhängig von der Lumenanzahl die Vorrichtung (1) hinsichtlich folgender Eigenschaft gesteuert wird:
Erkennung des korrekten Schlauchsystems.

2. Verfahren gemäß Anspruch 1, **dadurch gekennzeichnet, dass** der zu kontrollierende Abschnitt ein Schlauchsystem ist, das von dem Gehäuseteil der Vorrichtung zu dem Körper führt.

3. Verfahren gemäß Anspruch 1 oder 2, **dadurch gekennzeichnet, dass** die Druckveränderung eine einstufige Druckveränderung ist, bevorzugt ausgewählt ist aus der Gruppe bestehend aus linearer Druckveränderung, Einsprungfunktion, kurvenförmiger Druckveränderung, wie sinusförmig oder exponentiell, und besonders bevorzugt eine Druckerniedrigung ist.

4. Verfahren gemäß Anspruch 1 oder 2, **dadurch gekennzeichnet, dass** die Druckveränderung eine pulsförmige Druckveränderung ist, bevorzugt ausgewählt ist aus der Gruppe bestehend aus Rechteckpuls, Dreieckpuls, Sägezahnpuls, sinusförmiger Puls, und besonders bevorzugt eine pulsförmige Druckerniedrigung ΔP ist.

5. Verfahren gemäß Anspruch 4, **dadurch gekennzeichnet, dass** Druckveränderungspuls eine Pulsdauer von zwischen 5 und 500 ms, bevorzugt von zwischen 10 und 200 ms, besonders bevorzugt von zwischen 15 und 100 ms, und insbesondere bevorzugt von zwischen 20 und 40 ms aufweist.

6. Verfahren gemäß einer der vorherigen Ansprüche, **dadurch gekennzeichnet, dass** die einstufige oder pulsförmige Druckerniedrigung durch transiente Öffnung eines Ventils erfolgt, das bevorzugt distal der Drucksensoren an das Fluidsystem angeschlossen ist.

7. Verfahren gemäß einem der vorangehenden Ansprüche, **dadurch gekennzeichnet, dass** das die Druckveränderung ΔP, die bevorzugt eine Druckerniedrigung ist, einen Wert zwischen 0,01 mmHg und 300 mmHg aufweist.

8. Unterdrucktherapievorrichtung zur Durchführung eines Verfahrens gemäß einem der Ansprüche 1 bis 7, umfassend:
(a) eine Unterdruckpumpe,
(b) optional ein Aufnahmebehälter für Flüssigkeiten,
(c) mindestens zwei Drucksensoren,
(d) Steuerbares Belüftungsventil,
(e) ein Anschlussmittel, das sowohl den Anschluss eines einlumigen als auch eines zweilumigen oder mehrlumigen Schlauchsystems ermöglicht, und
(f) eine elektronische Steuerungseinheit, die an die Unterdruckpumpe, das Belüftungsventil und an die zwei Drucksensoren anschließbar ist,
wobei
die Steuerungseinheit derart ausgebildet ist, dass sie über die Ansteuerung des Belüftungsventils und Empfangen der Signale der mindestens zwei Drucksensoren die Lumenanzahl eines an dem Anschlussmittel angeschlossenen Schlauchsystems erkennt und dass sie im Erkennungsfall den Betrieb der Vorrichtung an die Lumenanzahl des Schlauchsystems anpasst.

9. Computerprogrammprodukt, das direkt in eine Speichereinheit geladen werden kann und Softwareabschnitte umfasst, mit denen das Verfahren gemäß einem der Ansprüche 1 bis 7 ausgeführt werden kann, wenn das Computerprogrammprodukt auf einer Vorrichtung gemäß Anspruch 8 ausgeführt wird.

## Claims

1. Method for checking a portion in fluid systems of a negative pressure therapy system in order to identify the correct tubing system, wherein this portion is delimited by two pressure sensors (111, 121), the method comprising the following steps:
(a) building up a negative pressure in the portion of the fluid system to be checked;
(b) generating a pressure change in the fluid system;
(c) detection of the pressure change by the two pressure sensors (111, 121);
(d) evaluating the pressure change detected by the sensors (111, 121) with regard to its time difference and/or its magnitude and/or its form;
(e) controlling a device for negative pressure therapy (1) depending on the evaluation performed in step (d),
wherein
the length of the portion in the fluid system can be detected by the time difference measured in method step (c) or by the pressure difference in a predetermined time difference between the measurement of the two pressure sensors, and
the number of lumen of the tubing system can be detected by the time difference or pressure difference measured in method step (c), and
wherein
depending on the number of lumen, the device (1) is controlled with respect to the following property:
identification of the correct tubing system.

2. Method according to claim 1, **characterized in that** the portion to be checked is a tubing system which leads from the housing part of the device to the body.

3. Method according to either claim 1 or 2, **characterized in that** the pressure change is a single-stage pressure change, preferably selected from the group consisting of linear pressure change, jump function, curved pressure change, such as sinusoidal or exponential, and particularly preferably a pressure reduction.

4. Method according to either claim 1 or 2, **characterized in that** the pressure change is a pulse-shaped pressure change, preferably selected from the group consisting of rectangular pulse, triangular pulse, sawtooth pulse, sinusoidal pulse, and particularly preferably a pulse-shaped pressure reduction ΔP.

5. Method according to claim 4, **characterized in that** pressure-changing pulse has a pulse duration of between 5 and 500 ms, preferably of between 10 and 200 ms, particularly preferably of between 15 and 100 ms, and in particular preferably of between 20 and 40 ms.

6. Method according to any of the preceding claims, **characterized in that** the single-stage or pulse-shaped pressure reduction takes place by transient opening of a valve, which is preferably connected to the fluid system distally of the pressure sensors.

7. Method according to any of the preceding claims, **characterized in that** the pressure change ΔP, which is preferably a pressure decrease, has a value between 0.01 mmHg and 300 mmHg.

8. Negative pressure therapy device for carrying out a method according to any of claims 1 to 7, comprising:
(a) a vacuum pump,
(b) optionally a receptacle for liquids,
(c) at least two pressure sensors,
(d) controllable ventilation valve,
(e) a connection means, which allows the connection of both a single-lumen as well as a double-lumen or multi-lumen tubing system, and
(f) an electronic control unit, which can be connected to the vacuum pump, the ventilation valve and to the two pressure sensors,
wherein
the control unit is designed such that it identifies the number of lumens of a tubing system connected to the connection means via the activation of the ventilation valve and receiving the signals of the at least two pressure sensors, and that, in the event of identification, it adapts the operation of the device to the number of lumen of the tubing system.

9. Computer program product that can be loaded directly into a memory unit and comprises software portions by means of which the method according to any of claims 1 to 7 can be carried out when the computer program product is executed on a device according to claim 8.

## Revendications

1. Procédé permettant de contrôler une section dans des systèmes fluidiques d'un système de thérapie par pression négative pour la détection du système de tubulures correct, dans lequel ladite section est délimitée par deux capteurs de pression (111, 121), comprenant les étapes suivantes :
(a) établissement d'une pression négative dans la section à contrôler du système fluidique ;
(b) génération d'une modification de pression dans le système fluidique ;
(c) détection de la modification de pression par les deux capteurs de pression (111, 121) ;
(d) évaluation de la modification de pression détectée par les capteurs (111, 121) en ce qui concerne sa différence temporelle et/ou sa grandeur et/ou sa forme ;
(e) commande d'un dispositif pour la thérapie par pression négative (1) en fonction de l'évaluation réalisée à l'étape (d),
dans lequel
au moyen de la différence temporelle mesurée à l'étape de procédé (c) ou au moyen de la différence de pression dans le cas d'une différence temporelle prédéfinie entre la mesure des deux capteurs de pression, la longueur de la section dans le système fluidique peut être détectée, et
au moyen de la différence temporelle ou de pression mesurée à l'étape de procédé (c), le nombre de lumières du système de tubulures peut être détecté, et
dans lequel
en fonction du nombre de lumières, le dispositif (1) est commandé en ce qui concerne la propriété suivante :
identification du système de tubulures correct.

2. Procédé selon la revendication 1, **caractérisé en ce que** la section à contrôler est un système de tubulures qui va de la partie de boîtier du dispositif jusqu'au corps.

3. Procédé selon la revendication 1 ou 2, **caractérisé en ce que** la modification de pression est une modification de pression en une étape, de préférence est choisie dans le groupe constitué d'une modification de pression linéaire, d'une fonction de saut unique, d'une modification de pression en forme de courbe, telle que sinusoïdale ou exponentielle et, de manière particulièrement préférée, d'une diminution de pression.

4. Procédé selon la revendication 1 ou 2, **caractérisé en ce que** la modification de pression est une modification de pression sous forme d'impulsion, de préférence est choisie dans le groupe constitué d'une impulsion rectangulaire, d'une impulsion triangulaire, d'une impulsion en dents de scie, d'une impulsion sinusoïdale et, de manière particulièrement préférée, d'une diminution de pression en forme d'impulsion ΔP.

5. Procédé selon la revendication 4, **caractérisé en ce que** impulsion de modification de pression présente une durée d'impulsion comprise entre 5 et 500 ms, de préférence comprise entre 10 et 200 ms, de manière particulièrement préférée comprise entre 15 et 100 ms et, de manière tout particulièrement préférée, comprise entre 20 et 40 ms.

6. Procédé selon l'une des revendications précédentes,
**caractérisé en ce que** la diminution de pression en une étape ou sous forme d'impulsion a lieu par l'ouverture transitoire d'une soupape qui est de préférence raccordée au système fluidique de manière distale par rapport aux capteurs de pression.

7. Procédé selon l'une des revendications précédentes,
**caractérisé en ce que** la modification de pression ΔP, laquelle est de préférence une diminution de pression, présente une valeur comprise entre 0,01 mm de Hg et 300 mm de Hg.

8. Dispositif de thérapie par pression négative permettant de réaliser un procédé selon l'une des revendications 1 à 7, comprenant :
(a) une pompe à vide,
(b) éventuellement un récipient de réception pour liquides,
(c) au moins deux capteurs de pression,
(d) une soupape d'aération pouvant être commandée,
(e) un moyen de raccordement, lequel permet le raccordement d'un système de tubulures à une lumière ainsi que d'un système de tubulures à deux lumières ou à plus de lumières, et
(f) une unité de commande électronique, laquelle peut être raccordée à la pompe à vide, à la soupape d'aération et aux deux capteurs de pression,
dans lequel
l'unité de commande est configurée de manière telle qu'elle détecte, par l'intermédiaire de la commande de la soupape d'aération et de la réception des signaux des au moins deux capteurs de pression, le nombre de lumières d'un système de tubulures raccordé au moyen de raccordement et qu'elle adapte le fonctionnement du dispositif au nombre de lumières du système de tubulures en cas de détection.

9. Produit programme informatique, lequel peut être chargé directement dans une unité de stockage et comprend des sections logicielles à l'aide desquelles le procédé selon l'une des revendications 1 à 7 peut être exécuté lorsque le produit programme informatique est exécuté sur un dispositif selon la revendication 8.
